(19) Europäisches Patentamt
European Patent Office
Office européen des brevets

(11) Veröffentlichungsnummer: **0 013 405**
**B1**

# (12) EUROPÄISCHE PATENTSCHRIFT

(45) Veröffentlichungstag der Patentschrift: **08.12.82**

(51) Int. Cl.³: **C 12 P 33/06,** C 07 J 7/00, C 07 J 53/00

(21) Anmeldenummer: **79105316.8**

(22) Anmeldetag: **21.12.79**

(54) **Verfahren zur Herstellung von 19-Hydroxysteroiden der Androstan- und Pregnanreihe.**

(30) Priorität: **12.01.79 DE 2901564**

(43) Veröffentlichungstag der Anmeldung: **23.07.80 Patentblatt 80/15**

(45) Bekanntmachung des Hinweises auf die Patenterteilung: **08.12.82 Patentblatt 82/49**

(84) Benannte Vertragsstaaten: **AT BE CH DE FR GB IT LU NL SE**

(56) Entgegenhaltungen:
**CH-A-368 796**
**FR-A-1 281 867**
**FR-A-1 586 869**
**US-A-3 039 926**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Petzoldt, Karl, Dr., Flachsweg 10, D-1000 Berlin 38 (DE)**

EP 0 013 405 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 19-Hydroxysteroiden der Androsten- und Pregnanreihe

Die Erfindung betrifft das in den Patentansprüchen gekennzeichnete Verfahren.

19-Hydroxysteroide sind bekanntlich wichtige Zwischenprodukte zur Partialsynthese pharmakologisch wirksamer 19-Norsteroide. Ihre chemische Synthese aus den entsprechenden 10-Methylsteroiden ist vielstufig und sehr aufwendig (J. Amer. Chem. Soc., 84, 1962, 3204 f und J. Amer. Chem. Soc., 83, 1961, 4076 f). Es wurden auch bereits Untersuchungen durchgeführt, 10-Methylsteroide mittels Mikroorganismen-Kulturen in der 19-Position zu hydroxylieren (Bull. Agr. Chem. Japan 22, 1958, 212), die bisher bekannten Verfahren liefern aber nur so geringe Ausbeuten an 19-Hydroxysteroiden, daß sie technisch nicht anwendbar sind.

Demgegenüber ist es mit Hilfe des erfindungsgemäßen Verfahrens möglich, 10-Methylsteroide in hoher Ausbeute in die entsprechenden 19-Hydroxysteroide umzuwandeln.

Das erfindungsgemäße Verfahren wird unter den Bedingungen durchgeführt, die man üblicherweise bei der mikrobiologischen Hydroxylierung von Steroiden mit Pilzkulturen anwendet. So werden zunächst in allgemein üblichen Vorversuchen die günstigsten Fermentationsbedingungen, wie zum Beispiel Auswahl des günstigsten Nährmediums, des geeigneten Substratlösungs- oder Suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt. Dabei hat sich gezeigt, daß es zweckmäßig ist, Konzentrationen von etwa 100—2000 mg Substrat pro Liter Nährmedium einzusetzen. Der pH-Wert wird vorzugsweise auf einen Wert im Bereich von 5—7 eingestellt. Die Züchtungstemperatur liegt im Bereich von 20—40°C, vorzugsweise von 25—35°C. Zur Belüftung werden vorzugsweise 0,5 bis 5 Liter Luft pro Minute pro Liter Kulturbrühe zugeführt. Die Umwandlung des Substrates wird zweckmäßigerweise durch dünnschichtchromatographische Analyse von Probeextrakten verfolgt. Die Fermentationsdauer beträgt etwa 90 bis 100 Stunden.

Nach erfolgter Fermentation werden die Fermentationsprodukte in an sich bekannter Weise isoliert. Die Isolierung kann zum Beispiel in der Weise erfolgen, daß man die Fermentationsansätze mit einem nicht mit Wasser mischbaren organischen Lösungsmittel wie Äthylacetat, Butylacetat oder Methylisobutylketon, extrahiert, die Extrakte einengt und die so erhaltenen Rohprodukte gegebenenfalls durch Chromatographie und/oder Kristallisation reinigt.

Die als Ausgangsverbindungen für das erfindungsgemäße Verfahren benötigten 10-Methylsteroide können in üblicher Weise substituiert sein. Geeignete Substituenten sind beispielsweise in der 3β,11β,17α,17β und/oder 21-Stellung ständige Hydroxygruppen oder deren Ester von Alkancarbonsäuren mit 1 bis 8 Kohlenstoffatomen, in der 3,11,17 und/oder 20-Position ständige Oxogruppen, in der 6 und/oder 9-Position ständige Halogenatome (vorzugsweise Fluoratome oder Chloratome) in der 6 und/oder 16-Stellung ständige Methylgruppen und/oder in der 1α,2α-, 15α,16α-, 15β,16β-Position befindliche Methylengruppen. Die 10-Methylkortikoide können gesättigt oder beispielsweise in der 1,2-, 4,5-, 5,6-, 9,11- und/oder 15,16-Position ungesättigt sein.

Bevorzugte 10-Methylsteroide sind solche der allgemeinen Formel II, welche als Substituenten R beispielsweise ein Wasserstoffatom, eine Formyloxygruppe, eine Propionyloxygruppe oder Butyryloxygruppe tragen können.

Zur Fermentation wird eine Pilzkultur der Species Nigrospora sphaerica zum Beispiel ATCC 12 772 verwendet.

Die nachfolgenden Ausführungsbeispiele dienen zur Erläuterung des erfindungsgemäßen Verfahrens:

## Beispiel 1

Ein 2l-Erlenmeyerkolben, der 500 ml einer 30 Minuten bei 120°C im Autoklaven sterilisierten Nährlösung aus 3% Glukose, 1% Corn steep, 0,2% $NaNO_3$, 0,1% $KH_2PO_4$, 0,2% $K_2HPO_4$, 0,05% $MgSO_4 \cdot 7\,H_2O$, 0,002% $FeSO_4 \cdot 7\,H_2O$ und 0,05% KCl enthält, wird mit einer Lyophilkultur von Nigrospora sphaerica beimpft und 72 Stunden bei 30°C auf einem Rotationsschüttler geschüttelt. Diese Anzuchtskultur dient zur Beimpfung eines 10l-Vorfermenters, der mit 7,5 l eines bei 121°C und 1,1 atü sterilisierten Nährmediums der gleichen Zusammensetzung wie die Anzuchtkultur beschickt ist. Unter Zugabe von Silicon SH als Antischaummittel wird nun bei 29°C und 0,7 atü Druck unter Belüftung (10 l/Min.) und Rühren (220 U/min.) 36 Stunden germiniert. Danach werden 1,5 l dieser Kultur unter sterilen Bedingungen entnommen und damit ein 30l-Hauptfermenter beimpft, der mit 23,5 l eines wie oben sterilisierten Nährmediums, bestehend aus 1% Cornsteep und 1,25% Sojabohnenmehl, eingestellt auf pH 6,2, gefüllt ist. Nach einer Anwachsphase von 12 Stunden unter Vorfermenterbedingungen werden 12,5 g Cyproteron (6-Chlor-17α-hydroxy-1α,2α-methylen-4,6-pregnadien-3,20-dion), gelöst in 300 ml Dimethylformamid, unter sterilen Bedingungen hinzugegeben und weiter gerührt und belüftet. Der Verlauf der Fermentation wird durch Entnahme von Proben kontrolliert, die mittels Methylisobutylketon estrahiert und dünnschichtchromatographisch analysiert werden. Nach 72 Stunden Kontaktzeit ist die Umsetzung

des Substrates beendet. Der Fermenterinhalt wird zweimal mit je 20 l Methylisobutylketon extrahiert, die Extrakte vereinigt und zunächst im Umlaufverdampfer konzentriert, anschließend bei 50°C Badtemperatur im Vakuum im Rotationsverdampfer zur Trockne eingeengt. Den Rückstand löst man in warmem Methanol, filtriert vom ungelöst gebliebenen Siliconöl ab, behandelt das Filtrat mit Aktivkohle und dampft wieder zur Trockne ein. Der verbliebene Rückstand (15 g) wird schließlich aus Aceton/Diisopropyläther kristallisiert. Man erhält 8,8 g 6-Chlor-17α,19-dihydroxy-1α,2α-methylen-4,6-pregnadien-3,20-dion vom Schmelzpunkt 215—216°C.

Beispiel 2

Unter den Bedingungen des Beispiels 1 werden 10 g Megestrol (17α-Hydroxy-6-methyl-4,6-pregnadien-3,20-dion), gelöst in 400 ml Dimethylformamid, mit einer Nigrospora sphaerica-Kultur 48 Stunden fermentiert. Nach Aufarbeitung des Ansatzes und Kristallisation des Rohproduktes aus Aceton/Diisopropyläther erhält man 6,5 g 17α,19-Dihydroxy-6-methyl-4,6-pregnadien-3,20-dion vom Schmelzpunkt 184—186°C.

Beispiel 3

1 g 19-Hydroxy-cyproteron (6-Chlor-17α,19-dihydroxy-1α,2α-methylen-4,6-pregnadien-3,20-dion) wird in 7,5 ml Essig gelöst, 3 ml Trifluoracetanhydrid sowie 150 mg p-Toluolsulfonsäure zugegeben und 15 Minuten bei Raumtemperatur gerührt. Danach wird das Reaktionsgemisch in Eiswasser eingegossen, der ausgefallene Niederschlag abgesaugt, neutral gewaschen und getrocknet. Zur Auftrennung des Rohproduktgemisches chromatographiert man über eine Kieselgelsäule und eluiert mittels des Lösungsmittelgradienten Methylenchlorid-Methylenchlorid/Aceton (8+2):

a) Die zuerst aus der Säule austretende Fraktion I wird im Vakuum zur Trockne eingedampft und aus Aceton/Diisopropyläther kristallisiert. Man erhält 560 mg 17α,19-Diacetoxy-6-chlor-1α,2α-methylen-4,6-pregnadien-3,20-dion vom Schmelzpunkt 248—250°C.
b) Die anschließend erscheinende Fraktion II wird in gleicher Weise eingeengt und aus Aceton/Diisopropyläther zu 324 mg 17α-Acetoxy-6-chlor-19-hydroxy-1α,2α-methylen-4,6-pregnadien-3,20-dion vom Schmelzpunkt 220—221°C kristallisiert.

## Patentansprüche

1. Verfahren zur Herstellung von 19-Hydroxysteroiden der Androstan- und Pregnanreihe, dadurch gekennzeichnet, daß man ein entsprechendes 10-Methylsteroid mit einer Pilzkultur vom Species Nigrospora sphaerica fermentiert.

2. Verfahren zur Herstellung von 19-Hydroxysteroiden der allgemeinen Formel I

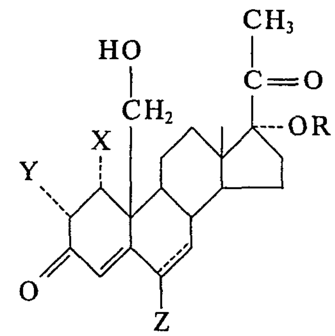

(I)

worin

........ eine Einfachbindung oder eine Doppelbindung,
X und Y jeweils Wasserstoffatome oder gemeinsam eine Kohlenstoff-Kohlenstoff-Bindung oder eine Methylengruppe,
Z ein Wasserstoffatom, ein Fluoratom, ein Chloratom oder eine Methylgruppe und
R ein Wasserstoffatom oder eine 1 bis 8 Kohlenstoffatome enthaltende Acyloxygruppe bedeuten,

dadurch gekennzeichnet, daß man ein 10-Methylsteroid der allgemeinen Formel II

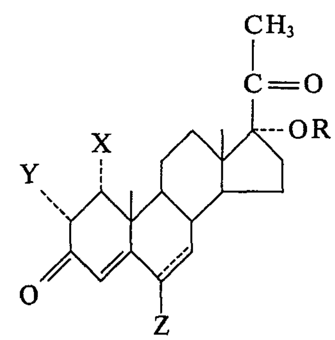

(II)

worin
......, X, Y, Z und R die obengenannte Bedeutung besitzen, mit einer Pilzkultur der Species Nigrospora sphaerica fermentiert.

## Claims

1. Process for the manufacture of 19-hydroxysteroids of the androstane and pregnane series, characterised in that a corresponding 10-methylsteroid is fermented with a fungal culture of the species Nigrospora sphaerica.

2. Process for the manufacture of 19-hydroxysteroids of the general formula I

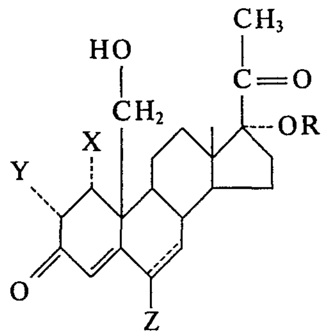

(I)

in which

....... represents a single bond or a double bond,

X and Y each represents a hydrogen atom or together represent a carbon-carbon bond or a methylene group,

Z represents a hydrogen atom, a fluorine atom, a chlorine atom or a methyl group, and

R represents a hydrogen atom or an acyloxy group containing from 1 to 8 carbon atoms,

characterised in that a 10-methylsteroid of the general formula II

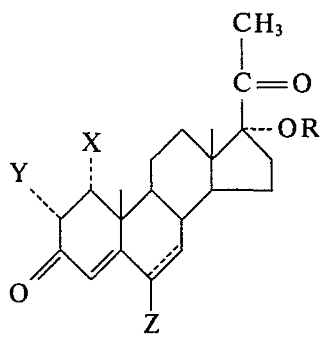

(II)

in which

.......,X, Y, Z and R have the meanings given above is fermented with a fungal culture of the species Nigrospora sphaerica.

**Revendications**

1. Procédé pour la production des 19-hydroxystéroïdes de la série des androstanes et des prégnanes, caractérisé en ce que l'on fait fermenter un 10-méthylstéroïde homologue avec une culture de champignon de l'espèce Nigrospora sphaerica.

2. Procédé pour la production de 19-hydroxystéroïdes de formule générale I

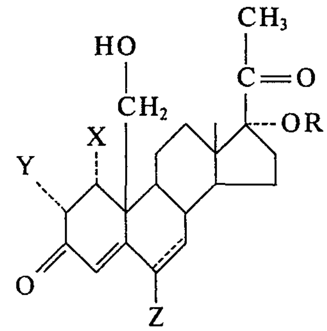

(I)

dans laquelle

....... représente une liaison simple ou double,

X et Y représentent chacun un atome d'hydrogène ou ensemble une liaison ou un groupe méthyle,

Z représente un atome d'hydrogène, de fluor, de chlore ou un groupe méthyle, et

R représente un atome d'hydrogène ou un groupe acyloxy ayant de 1 à 8 atomes de carbone,

caractérisé en ce que l'on fait fermenter avec une culture de chanpignon de l'espèce Nigrospora sphaerica un 10-méthylstéroïde de formule générale II

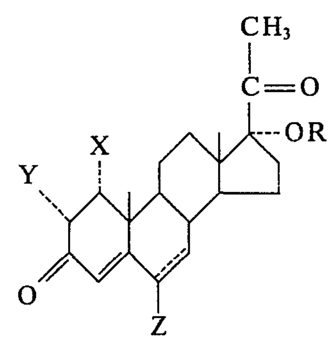

(II)

dans laquelle

.......,X, Y, Z et R ont les significations ci-dessus.